# EUROPEAN PATENT SPECIFICATION

(11) **EP 0 625 224 B1**
(45) Date of publication and mention of the grant of the patent: **24.04.1996**
(21) Application number: 93903210.8
(22) Date of filing: 20.01.1993
(51) Int. Cl.: D21H 21/54, G03F 3/10, G03C 8/24, G03C 1/775, G03C 1/795

(54) **IMAGE CARRIER FOR A REPROGRAPHIC IMAGE**
TRÄGER FÜR REPROGRAPHISCHE BILDER
SUPPORT D'IMAGES POUR IMAGE REPROGRAPHIQUE

(30) Priority: 04.02.1992 EP 92200305
(43) Date of publication of application: 23.11.1994
(73) Proprietor: AGFA-GEVAERT naamloze vennootschap, B-2640 Mortsel (BE)
(72) Inventor: LEENDERS, Luc, B-2640 Mortsel (BE); DAEMS, Eddie, B-2640 Mortsel (BE)
(86) International application number: EP9300128
(87) International publication number: WO9315272

(56) References cited:
- EP-A- 0 305 599
- US-A- 4 145 480
- US-A- 4 469 825
- US-A- 4 972 000
- DATABASE WPIL Week 9119, Derwent Publications Ltd., London, GB; AN 91-136965
- DATABASE WPIL Week 8846, Derwent Publications Ltd., London, GB; AN 88-327894

## Description

### IMAGE CARRIER FOR A REPROGRAPHIC IMAGE

### 1. Field of the invention.

The present invention relates to an image carrier for a reprographic image, e.g. a black-and-white reprographic image or a multicolour image intended as pre-press colour proof, and to processes for forming said images.

### 2. Background of the invention.

In the colour field of pre-press graphic arts there has been a long felt need for a simple and fast technique offering "colour proofs" of high quality and reproducibility.

Photographically produced colour proofs are a simulation for multicolour halftone reproductions as will be produced by successive printing in register with the separate standard inks : magenta, yellow, cyan and black on a conventional printing press.

Press proofing for the production of colour proofs by preparing a printing plate and running the plate on the press to produce only a few copies as proof of the quality of the halftone separation transparencies used in the plate production is a very cumbersome and expensive procedure and therefore photographic processes have been developed to obtain a similar result by means of which the appearance of a print starting from particular colour separation negatives or positives can be judged by the printer and client.

According to a process known from US-A-3,642,474 the production of such a proof in the form of superposed coloured colloid patterns, proceeds with coloured hydrophilic colloid layers, which are insolubilized in the irradiated portions by means of an active species formed during or after the information-wise exposure to active electromagnetic radiation of a photosensitive substance, and comprises the following steps :
(1) transferring a coloured hydrophilic colloid layer containing a said photosensitive substance and a hydrophilic colloid, which undergoes a reduction in solubility in water by said active species, from a temporary support, which is more hydrophobic than a permanent support to which said layer has to be transferred, to said permanent support by pressing the latter in the presence of an aqueous liquid against said colloid layer, and removing the temporary support, thus leaving said layer on the permanent support;
(2) exposing the transferred colloid layer in substantially dry state to actinic electromagnetic radiation, which is modulated according to the information to be recorded,
(3) developing the exposed layer by means of an aqueous liquid followed by a wash-off processing resulting in a coloured relief pattern, and repeating the steps (1), (2) and (3) with said hydrophilic colloid layers having a colour as desired to produce superposed coloured colloid patterns on the permanent support.

In said process for producing a multicolour pattern the exposures proceed in register on the same permanent support which received the unexposed coloured hardenable colloid layers by transfer from a temporary support, each transfer and exposure being followed by hardening development, wash-off processing and a drying step before a next transfer and exposure leading to a further selection colour pattern can be carried out.

In most of the examples of said US-A-3,642,474 an ultraviolet radiation sensitive iron(III) complex is used which yields iron(II) upon UV-irradiation forming by reaction with hydrogen peroxide hydroxyl radicals that imagewise harden the gelatin binder of each coloured layer. However said iron(III) complex, e.g. ammonium iron(III) oxalate, has a poor photosensitivity in comparison with silver halide which makes the method unsuitable for exposure by low radiation sources like the output lasers of modern scanners.

A process for producing a multicolour pattern using silver halide emulsion materials is described in EP-A- 0 185 410. This method comprises the following steps:
(1) the scanningwise exposure of a multicolour original attached to a rotating scanner drum in order to obtain separate red light, green light and blue light output signals received by photon-detectors to produce corresponding electrical signals, which are fed into a computer,
(2) the computer controlled exposure, of differently coloured hardening developable photographic materials each comprising on a temporary support one or more hardenable hydrophilic colloid layers at least one of which contains dispersed photosensitive silver halide, either
(3) transferring integrally said hydrophilic colloid layer(s) of a first of said exposed photographic materials onto a permanent support, which at its surface is less hydrophobic than the temporary support, by pressing the permanent support in the presence of an aqueous liquid against the hydrophilic colloid layer side of said photographic material and removing the temporary support, thus leaving said hydrophilic colloid layer(s) on said same permanent support, and
(4) developing the transferred exposed photosensitive silver halide with a hardening developing agent to form imagewise hardened coloured hydrophilic colloid portions and removing the non-hardened colloid portions to leave a coloured relief image on the permanent support, and
(5) producing a multicolour pattern on said same permanent support by repeating the steps (3) and (4) with one or more other scanning-wise exposed differently coloured photographic materials, the transfer procedure of step (3) being effected in image register.

However the direct exposure by the scanner output laser of the different coloured materials requires the presence of special silver halide emulsions spectrally sensitized to the emission wavelenght of the laser of the scanner used. A more simple and conventional procedure consists in generating by a scanner colour separation negatives or positives on a scan film and exposing the described coloured colloid materials through these separation negatives or positives in a conventional contact-apparatus provided with an ultraviolet rich radiation source. In this way rather insensitive non-spectrally sensitized emulsions rich in chloride can be used.

The permanent support mentioned in the cited application can be an organic polymeric resin support, e.g. a polyethylene terephtalate support, or it can be a paper base, e.g. a polyolefine covered paper base.

As is known a halftone image consists of numerous dots of different areas that are created when a continuous tone image is reproduced by exposure through a halftone screen, or obtained by halftone modulated scanning exposure. Due to a number of factors - some of which are controllable, others of which are not - the dot increases in size from the time it is first created on the photographic material serving as intermediate original in the formation of the printing form until it is finally reproduced as an ink dot in the printing step. A certain percentage of so-called "dot gain" is obtained, which has to be taken into account in the production of colour proofs which should ressemble as close as possible the prints made on the press.

Dot gain in printing is made up of two components, physical and optical dot gain. Physical or mechanical dot gain is the actual physically measurable enlargement of the dot size which occurs during the printing process wherein individual ink dots are spread on the printing paper. The percentage of mechanical dot gains depends on different parameters including paper stock quality, press setting, press conditions and nature of printing plate.

Optical dot gain results when light penetrates the printing paper in the clear areas, becomes internally scattered therein and partially absorbed in the ink dots making the dots visually appear larger. Optical dot gain can be influenced by changing the paper stock (see the article "Gaining on Dot Gain" by Johan Strashun in Graphic Arts Montly, January 1985, p. 69).

In offset printing the greatest dot gain occurs in the mid tones, i.e. around the 50 percent dot value. Good colour balance is obtained when all colours have equal dot gain.

In simulating the printing result with a colour proof it is important to obtain a total dot gain in the colour proof as close as possible to the total dot gain obtained in the actual print, whatever the printing stock or printing method may be. So it would be advantageous to dispose of a set of different permanent supports on wich by successive transfer of coloured colloid layers colour proofs can be generated showing controlable but mutually different dot gains.

Such a set of different supports, in particular paper bases, giving rise to different dot gains and the process for producing multicoloured colloid patterns thereon, are disclosed in European patent application EP 0 305 599. The disclosed supports essentially comprise a polyolefine covered paper base coated with a hydrophylic binder layer containing mutually different concentrations of titanium dioxide particles. These different concentrations cause different light-scattering properties resulting in different dot gains in the final colour proof.

Materials and apparatuses used in accordance with the methods for producing colour proofs disclosed in the applications EP 0 185 410 and EP 0 305 599 cited above are commercalized by AGFA-GEVAERT N.V. under the registered trade name AGFAPROOF system. A set of three permanent paper supports can be provided giving rise to finished proofs showing high, low or medium dot gain respectively.

In the practical application of the teachings of EP-A-0 305 599 a problem arises when the employed titanium dioxide is of the most common type namely the rutile crystal modification. By increasing the amount of titanium dioxide in the hydrophilic binder layer the dot gain percentage can be varied from high over medium to low. However increasing amounts of rutile titanium dioxide produce a more and more yellowish hue of the receptor element due to the fact that the rutile modification absorbs a minor amount of visible light in the spectral region around 400 nm and above. These different shifts of ideal white in the non-image parts of colour proofs, produced on different permanent supports containing different amounts of rutile titanium dioxide, influence the visual contrast and the appreciation of the colour rendition by the printer or customer comparing such colour proofs on different bases. The difficulty could be overcome by replacing the relative high amounts of rutile titanium dioxide, needed for obtaining medium or low dot gain, by the anatase titanium oxide modification wich shows a complete reflectance of visible light also around 400 nm and as a consequence produces no yellowish hue. It was however established experimentally that receptor elements containing high amounts of anatase titanium dioxide in their hydrophilic binder layer, contrary to those containing high amounts of the rutile modification, are not able to give rise to a sufficiently low dot gain in the final proof (see control examples furtheron). As a consequence the anatase modification cannot be used as the only opacifying agent in receptor elements intended to give rise to low dot gains.

The present invention constitutes a further improvement to the teachings of EP-A-0 305 599.

It is an object of the present invention to provide an image carrier for a line and/or halftone reprographic image, which is able to give rise to a low dot gain and/or small line enlargement without disturbing deviation of ideal whiteness in the non-image parts of the final image. This reprographic image is preferably a multicolour colloid image serving as a pre-press proof or for other applications a black-and-white reprographic image.

It is a further object of the present invention to provide a set of different image carriers which are mutually different in their dot gain properties but show essentially the same background whiteness.

It is still a further object of the present invention to provide a process of producing on said image carriers a line and/or halftone reprographic image, preferably a multicolour image serving as a pre-press proof or alternatively a black-and-white image.

Further objects of the present invention will become clear from the description hereafter.

### 3. Summary of the invention.

The object of providing a carrier for a line and/or halftone reprographic image showing low dot gain and/or small line enlargement is realized in the present invention by providing an image carrier comprising a support and an opaque hydrophilic colloid binder layer, characterized in that said opaque hydrophilic colloid binder layer contains at least two kinds of opacifying agents, being
(a) polymeric particles consisting of hollow spherical core/sheat beads,
(b) titanium dioxide of the anatase crystal modification.

It was found surprisingly that by using sufficiently high amounts and a selected ratio of agents (a) and (b), the optical dot gain and line enlargement could be kept minimal while maintaining an excellent visual whiteness in the non-image parts.

The object of the present invention of providing a set of different image carriers, which are mutually different in their dot gain properties but show essentially the same background whiteness, was realized by mixing appropriate quantities of three opacifying agents being (a) polymeric particles consisting of hollow spherical core/sheat beads, (b) anatase titanium dioxide and (c) common rutile titanium dioxide. It was found that by varying the relative amounts of these three opacifying agents it was possible to vary both the whiteness and the dot gain properties independently within rather broad limits. Moreover by selecting optimal ratios of the amounts of these three opacifyers it was possible to provide a set of image carriers mutually different in their dot gain but showing the same background whiteness.

The image carrier of the present invention can be used in principle as permanent support for any colour proofing system. In a preferred embodiment however the image carrier is meant to serve as a permanent receptor element for colloid layers transferred to it by the AGFAPROOF process mentioned above. Other possible applications of the receptor materials can be found in black-and-white repography e.g. as carriers of a halftone or line work image obtained e.g. by the diffusion transfer reversal (DTR) process.

### 4. Detailed description of the invention.

In a preferred embodiment the support of the impage carrier of the present invention is a paper base having preferably a thickness range corresponding to a weight per square meter between 50 g/m and 300 g/m. The paper base is preferably covered with a polyolefine coating, e.g. polyethylene or polypropylene, preferably at a coverage ranging between 5 g/m and 40 g/m. The polyolefine layer itself may contain titanium dioxide, either rutile or anatase or a mixture of them.

Alternatively the support of the receptor material can be an organic polymeric resin support, e.g. a polyethylene terephtalate support. This support may itself be opacified with titanium dioxide particles, either rutile or anatase or mixtures of them, preferably at a concentration between 10 and 35 weight percent in respect of the polyethylene terephtalate weight. It was stated experimentally that in this case extra low dot gain values could be obtained.

In order to assure a good adhesion of support and hydrophilic binder layer a corona discharge treatment or a subbing layer can be applied to the support.

The hydrophilic binder layer containing the opacifying agents is coated at a gelatin coverage preferably ranging from 0.5 g/m to 5 g/m. Any conventional coating technique can be used. Instead of coating the hydrophilic binder layer can be applied by a transfer from a temporary support. This can be effected as a transfer of a separate single layer or as transfer of a layer pack, e.g. a pack consisting of the first colour pigmented layer to be transferred and the hydrophilic binder layer itself.

Particular useful hollow spherical core/sheat beads are described in US-A-4,427,836, US-A-4,468,498 and US-A-4,469,825. A preferred material is manufactured by ROHM AND HAAS Co, and sold under the trade name ROPAQUE (e.g. ROPAQUE type no. OP-62 and OP-84). A particular useful type is described as a hollow sphere composed of a copolymer of acrylic acid and styrene. During drying of the hydrophilic binder layer the enclosed water diffuses out of the inner sphere leaving an inner air containing center which enhances the light-scattering properties due to the refraction index transition at the sheat/air interphase.

The synthesis of other types of hollow polymeric beads, and references on prior art hollow polymeric beads, are described in US-A-4,972,000. The reference also discloses paper coating compositions containing such beads and which also can contain mineral opacifying agents such as titanium dioxide. The reference however does not deal with the formation of reprographic images.

The ratio of the amount of spherical beads to the amount of anatase titanium dioxide is preferably comprised between 10/1 and 1/10 (weight/weight).

A set of preferred receptor elements giving rise to respectively high, medium and low dot gain contain in their respective hydrophilic binder layers following range of amounts of opacifying agents :
- a) high dot gain :
   between 0.4 and 1.6 g/m of rutile titanium dioxide, and no spherical beads (cfr. prior art) :
- b) medium dot gain :
   between 0.2 and 1.0 g/m of spherical beads, between 1.0 and 2.0 g/m of anatase titanium dioxide and between 0.1 and 0.5 g/m of rutile titanium dioxide (invention) ;
- c) low dot gain :
   between 1.0 and 3.0 g/m of spherical beads, and between 3.0 and 10 g/m of anatase titanium dioxide (invention) ;

The hydrophilic binder layer of the receptor element can contain various other additives. Preferably optical brightners are present which thanks to their fluorescence enhance the whiteness of the receptor material. A preferred optical brightner is represented by chemical formula (B-I).

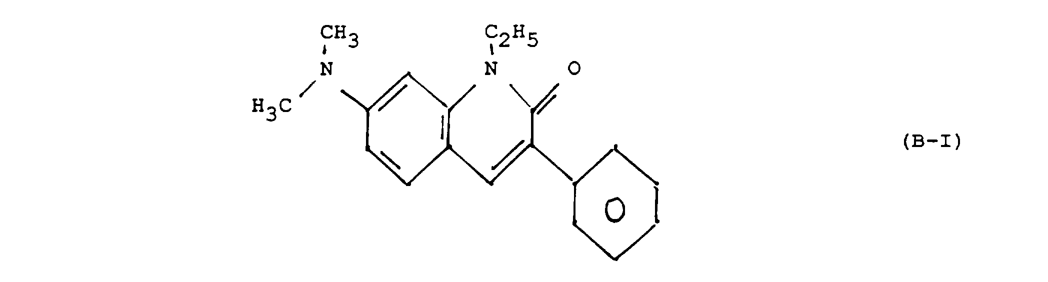

This optical brightner is preferably incorporated in the hydrophilic binder layer as loaded on a polymeric latex, e.g. a polyurethane latex.

The whiteness of the receptor element is quantitatively assessed by measurement with a GRETAG SMP 100 apparatus and expressed as L*, a*, b* values according to the CIE colorimetric system wherein a* and b* define the colour hue deviation from ideal white and L* is a measure for the luminance.

The colloid binding agent of the hydrophilic binder layer is preferably gelatin, preferably used in combination with a hydrophobic polymer in latex form, the hydrophobic polymer providing good adhesion of the titanium dioxide containing layer to the hydrophobic polyolefin layer upon its stratum formation during drying. A particularly useful latex polymer is copoly(methylmethacrylate-1,3 butadiene) (50/50 by weight) having a glass transition temperature (Tg) of 10 °C. The gelatin and latex polymer solids are preferably used in a weight ratio range of 1 : 5 to 5 : 1. The gelatin of the binder layer may be uniformly pre-hardened to some extent with a classical hardening agent therefore, e.g. formaldehyde.

The hydrophilic binder layer can further contain various spacing and/or matting agents, e.g. amorphous or crystalline silica particles, or hard polymeric beads, preferably showing an average particle size of 2 to 5 micron.

The support can be coated on one side with the described hydrophilic binder layer, or this layer can be applied on both sides.

The colour proofing process for which the image carrier of the present invention can serve as final permanent support can be of various nature. However in a preferred embodiment this process follows essentially the teachings of EP-A-0 305 599, cited above. This process essentially comprises the following steps :
(1) transferring a silver halide emulsion layer containing substantially non-hardened gelatin and coloured pigment particles dispersed therein from a temporary support onto an image carrier serving as receptor material,
(2) exposing information-wise the silver halide emulsion layer prior to or after carrying out said step (1),
(3) forming by hardening development a halftone image in said transferred silver halide layer and bleach-fixing said layer in order to remove developed silver and undeveloped silver halide,
(4) wash-off processing said transferred layer in order to remove selectively unhardened portions of said layer,
(5) repeating the previous steps in order to form at least partially superposed portions of differently coloured gelatin layers.

In this preferred embodiment the photosensitive compositions essentially comprise a temporary support, preferably a hydrophobic polymeric resin support, and at least one hydrophylic layer incorporating substantially unhardened gelatin as binder, a silver halide emulsion, and a dye pigment providing the desired colour. A silver halide hardening (tanning) developing agent and/or auxiliary developing agent can be incorporated in the layer or alternatively can be provided during processing in the developing solution.

In order to obtain transferred images with a good resolution, relatively thin hardenable coloured gelatin containing coatings are used. Preferably coatings having a thickness in the range of 1 micron and 15 micron are used, and good results are obtained with pigment coloured layers containing 1 to 10 g of gelatin/m.

Very good results from the viewpoint of image sharpness and mutual adherence of the differently coloured relief portions are obtained with a composite layer structure of a pigmented gelatin layer containing silver halide coated in combination with a non-pigmented gelatin top layer having a combined thickness preferably in the range of 1 to 3 micron. The thickness of the pigmented gelatin silver halide emulsion layer is preferably twice that of the non-pigmented gelatin silver halide emulsion top layer. The total gelatin coverage of said composite layer structure is preferably in the range of 1.0 to 3.0 g of gelatin/m. The pigmented gelatin containing silver halide emulsion layer contains preferably at least 50% by weight of gelatin. The non-pigmented layer preferably contains silver halide emulsion grains too in order to assure hardening during development.

The adhering power of said composite layer structure to its temporary support, preferably a flexible one, should be such that an easy stripping off from the temporary support is possible after pressing said composite layer structure into contact with a wetted receptor element serving as permanent support according to the present invention. The temporary support is e.g. an unsubbed cellulose triacetate sheet or a polyethylene terephtalate sheet covered with a subbing layer containing a Co(polyvinylacetate-crotonic acid, 85/15) copolymer as described in European patent application No. 92201839.5, filed June 23, 1992, and its corresponding US patent application serial no. 07/902,825, filed June 23, 1992. Other temporary supports having a repelling power for wet gelatin coatings are, e.g. a paper base coated with a polyethylene layer, a paper base impregnated with wax, a paper base coated with a layer of cellulose nitrate or a paper base coated with a layer of insolubilized polyvinyl alcohol or a layer of alginic acid insolubilized with an alkali earth metal salt.

The dye pigments are preferably used in diffusion-resistant form and can have all kinds of colour, e.g. are cyan, light-cyan, magenta, warm magenta, black, yellow, green, brown, orange, red, white or blue. Considered are likewise metallic colours such as pale gold, rich gold, copper, and silver. In other words the term "colour" in the present invention encompasses the use of all pure and mixed colours as well as black and white. In the standard procedure of making colour proofs simulating conventional four-colour printing photosensitive compositions containing respectively cyan, magenta, yellow and black pigments are used. In the production of superposed multicolour colloid patterns these standard pigments have to match with the spectral properties of the standard process inks as close as possible. Information about standard colour inks can be found in H.M.Cartwright - Ilford Graphic Arts Manual (1962) Vol. I - pages 502 to 504. For letterpress printing these standard colour inks have colour tones as defined in DIN 16538 and for offset printing the colour tones defined in DIN 16539. Further information about colour tones can be found for the USA in the GATF-Colour Charts.

It has been found experimentally that pigments, which are insoluble or very poorly soluble in water and in organic liquids of the alcohol or polyhydric alcohol type, e.g. glycerol, fulfil the requirements of resistance to diffusion. For colour proofing purposes a hardenable colloid layer in the present photographic material should preferably contain said pigments in a concentration sufficiently high for obtaining an optical density of at least 0.35 in the wavelength range of maximum absorption.

Pigments particularly suitable for use in the present invention are known organic non-migratory pigment type dyes, e.g. obtainable under the Trade Marks "HELIO-ECHT", "PIGMOSOL" and "COLANYL" dyes. "HELIO-ECHT", "PIGMOSOL" and "COLANYL" are diffusion resistant organic pigments that can be dispersed in aqueous medium with the aid of a dispersing agent. These pigments excel in resistance to light, heat, acids, bases, oxidizing agents, and solvents. They are insoluble in hydrophilic colloids such as gelatin.

When in addition to cyan, magenta and yellow relief patterns a black relief pattern is formed in colour proofing, preferably carbon black is used. Apart from carbon black mixtures of coloured pigments may be applied as described e.g. in US 4,427,757.

The photosensitive silver halide used in the silver halide emulsion layers of the photographic material used according to the present invention is e.g. silver chloride, silver bromide, silver bromoiodide, silver chlorobromoiodide, or mixtures thereof. The silver halide emulsions may be coarse or fine-grained and can be prepared by any of the well known procedures, e.g. as single jet or double jet precipitation technique.

When the photosensitive layers are exposed in a contact-exposure unit through colour separation negatives or positives serving as originals rather insensitive fine-grained and chloride rich emulsions can be used. Thes emulsions need no spectral sensitization since they are intrinsically sensitive to the UV-light of the exposure source. In the more particular application of direct scanning exposure of the photosensitive compositions appropriate spectral sensitization to the emission wavelength of the scanner laser is needed. In this case more sensitive emulsions must be used containing substantial amounts of bromide and/or iodide anions. For a proper spectral sensitization, e.g. with respect to laser beam light, the usual mono- or polymethine dyes such as acidic or basic cyanines, hemicyanines, oxonols, hemioxonols, styryl dyes or others, also tri- or polynuclear methine dyes, e.g. rhodacyanines or neocyanines may be used. Such spectral sensitizers are described, e.g., by F.M. HAMER in "The Cyanine Dyes and Related Compounds" (1964) Interscience Publishers, John Wiley & Sons, New York.

The silver halide emulsions can be of the conventional negative working surface-sensitive type, in particular when separation negatives are used as originals. In this case they can be chemically sensitized, e.g. by adding sulphur-containing compounds, e.g. allyl isothiocyanate, allyl thiourea, sodium thiosulphate and the like, during the chemical ripening stage. Also reducing agents, e.g. the tin compounds described in the Belgian Patent Specifications 493,464 and 568,687, and polyamines such as diethylenetriamine or derivatives of aminomethane-sulphonic acid, e.g. according to the Belgian Patent Specification 547,323, can be used as chemical sensitizers. Other suitable chemical sensitizers are noble metals and noble metal compounds such as gold, platinum, palladium, iridium, ruthenium and rhodium. This method of chemical sensitization has been described in the article of R.KOSLOWSKY, Z. Wiss. Photogr. Photophys. Photochem. 46, 65-72 (1951).

Alternatively the silver halide emulsions can be of the direct positive type, in particular when separation positives are used as originals. These direct positive emulsions can be externally prefogged and containing an electron acceptor, or they can be of the unfogged type containing internal electron traps and working with nucleating agents and fogging development ; in the latter case a nucleating agent can be present in at least one of the layers of the photosensitive composition or in the developing solution.

The silver halide emulsions may contain the usual stabilizers, e.g. homopolar or salt-like compounds of mercury with aromatic or heterocyclic rings such as mercaptotriazoles, simple mercury salts, sulphonium mercury double salts and other mercury compounds. Other suitable stabilizers are azaindenes, preferably tetra- or penta-azaindenes, especially those substituted with hydroxyl or amino groups. Compounds of this kind are described by BIRR in Z. Wiss. Photogr. Photophys. Photochem. 47,, 2-27 (1952). Still other suitable stabilizers are amongst others heterocyclic mercapto compounds, e.g. phenylmercaptotetrazole, quaternary benzothiazole derivatives and benzotriazole.

The hardening development of a latent silver image proceeds with commonly used hardening developing agents, also called tanning developing agents for effecting the development of the silver halide and producing oxidized developing agent acting as hardening agent for gelatin. Suitable hardening developing agents are :
1,4-dihydroxy benzene compounds such as hydroquinone,
chlorohydroquinone, bromohydroquinone, toluhydroquinone,
morpholinemethyl hydroquinone and sulfohydroquinone. Apart from the main hardening developing agent an auxiliary non-tanning developing agent can be used. Typical auxiliary developing agents include 3-pyrazolidinone developing agents, e.g. 1-phenyl-3-pyrazolidinone,
1-phenyl-4,4-dimethyl- 3-pyrazolidinone,
1-phenyl-4-methyl-4'-hydroxymethyl-3-pyrazolidinone and
N-methyl-p-aminophenol sulphate.

As stated above the developing agent(s) can be incorporated in the photosensitive silver halide material and/or in a developing bath. When incorporated in the photographic material it or they may be present in the silver halide emulsion layer or in a waterpermeable non-silver halide containing layer subjacent thereto, e.g. a top layer or an extra underlying layer. In this way several different combinations of the mutual location of hardening developing agent and auxiliary developing agent are possible. It is even possible that one of the developing agents, e.g. the main developing agent, is present in the developing solution while the other, e.g. the auxiliary developing agent is situated in one of the layers of the coloured photosensitive composition.

The silver halide emulsion layers used in accordance with the present invention may further comprise various kinds of surface-active agents in the photographic emulsion layer or in at least one other hydrophilic colloid layer. Suitable surface-active agents include non-ionic agents such as saponins, alkylene oxides e.g. polyethylene glycol, polyethylene glycol/polypropylene glycol condensation products, polyethylene glycol alkyl ethers or polyethylene glycol alkylaryl ethers, polyethylene glycol esters, polyethylene glycol sorbitan esters, polyalkylene glycol alkylamines or alkylamides, silicone-polyethylene oxide adducts, glycidol derivatives, fatty acid esters of polyhydric alcohols and alkyl esters of saccharides; anionic agents comprising an acid group such as a carboxy, sulpho, phospho, sulphuric or phosphoric ester group; ampholytic agents such as aminoacids, aminoalkyl sulphonic acids, aminoalkyl sulphates or phosphates, alkyl betaines, and amine-N-oxides; and cationic agents such as alkylamine salts, aliphatic, aromatic, or heterocyclic quaternary ammonium salts, aliphatic or heterocyclic ring-containing phosphonium or sulphonium salts. Such surface-active agents can be used for various purposes e.g. as coating aids, as compounds preventing electric charges, as compounds improving slidability, as compounds facilitating dispersive emulsification, as compounds preventing or reducing adhesion. Preferred surface-active agents are compounds containing perfluorinated alkyl groups.

The silver halide emulsion layers used in accordance with the present invention may further comprise various other additives such as e.g. compounds improving the dimensional stability of the photographic element, antistatic agents, spacing agents and UV-absorbers. Suitable additives for improving the dimensional stability of the photographic element are e.g. dispersions of a water-insoluble or hardly soluble synthetic polymer e.g. polymers of alkyl(meth)acrylates, alkoxy(meth)acrylates, glycidyl (meth)acrylates, (meth)acrylamides, vinyl esters, acrylonitriles, olefins, and styrenes, or copolymers of the above with acrylic acids, methacrylic acids, Alpha-Beta-unsaturated dicarboxylic acids, hydroxyalkyl (meth)acrylates, sulphoalkyl (meth)acrylates, and styrene sulphonic acids.

Another possible cause for bad registering is shrinkage of the paper base during each processing cycle. It was established experimentally that the shrinkage is minimal in the direction of the paper fibre. Therefore it is advantageous to assure that the receptor element containing one or more transferred coloured layers is introduced in the processing apparatus with the paper fibre direction coinciding with the processing direction. In order to indicate unambiguously the paper fibre direction a message can be applied at the back side of the receptor element, e.g. a printed figure or text.

Finally the coating composition(s) out of which the photosensitive element is coated preferably contains a water attracting compound, e.g. glycerol to give the coating a sufficient adherence to its temporary support and to enable an easy wet stripping off from the temporary support.

The exposure of each coloured photosensitive element can proceed before or after its transfer to the receptor element. For practical reasons, e.g. avoiding register problems, it is preferred to perform the exposure after transfer and short drying of each transferred coloured layer.

The successive transfer of each differently coloured photosensitive element can be carried out in an apparatus, in which the materials involved are pressed together between rollers in the presence of an aqueous liquid and peeling off the temporary support.

After each transfer a processing cycle is performed comprising a development step, bleach-fixing, a wash-off step and drying.

In case the developing agent(s) are present in the photosensitive element itself the development is carried out with a so-called activator liquid being an aqueous alkaline solution substantially free from developing agents. Typical activator liquids comprise, for example, an aqueous solution of an alkaline material, such as sodium carbonate, sodium hydroxide, potassium carbonate, potassium hydroxide, mixtures of sodium hydroxide and sodium sulfite, organic alkaline substances, e.g. alkanolamines, etc. A suitable activator bath comprises e.g. about 2 percent by weight of sodium hydroxide and 0.5 percent by weight of sodium sulphite.

Other adjuvants well known to those skilled in the art may be added to the developer or activator liquid used in accordance with the present invention. A survey of conventional developer addenda is given by Grant Haist in "Modern Photographic Processing" - John Wiley ans Sons - New York (1979) p. 220-224. Examples of such addenda include complexing agents for calcium and magnesium ions, present in hard water, e.g. ethylene diamine tetraacetic acid and analogues compounds. Further can be present anti-foaming agents, surface-active agents, biocides, thickening agents like polystyrene sulphonate and antioxidants like benzoate and cyclodextrine. The developing liquid can contain so-called anti-sludge agents in order to reduce dirt streaks on developed material.

In order to obtain pure colours the development step is followed by a bleach-fixing step wherein the developed metallic silver is bleached and removed together with residual undeveloped silver halide. The bleach-fixing bath preferably contains conventional fixing agents like sodium or ammonium thiosulphate, and conventional bleaching agents like complexes of iron(III) and polyaminocarboxylic acids, e.g. iron(III)-ethylenediamine-tetraacetic acid mono sodium salt.

In the following step the non-hardened portions of the hardening developed layers on the permanent support are washed off without mechanical rubbing by means of running water at a temperature preferably between 35 and 50°C. Excess of liquid is preferably removed by squeezing the relief between two smooth soft rollers, e.g. rubber rollers. Finally the whole composition is dried.

In a preferred procedure a multicolour printing proof is made according to the principles of subtractive colour photography starting with the production of a first relief image being a cyan part image of the multicolour original. In successive order a magenta, yellow and black relief image is produced on the same support. However, the order wherein the colour relief images are made can be chosen arbitrarily. After each transfer the processing cycle is repeated.

The obtained multicolour relief image can be protected by a transparent resin topcoat, which according to a preferred embodiment is applied by spraying a solution of film forming resin onto the relief image and drying. A suitable "spray-cover" consists of polyisobutyl- methacrylate.

The use of the image carrier of the present invention has been extensively described above in the context of the AGFAPROOF system where it is used as receptor material for the trasferred coloured layers. However it is specifically contemplated that its use can be extended to other colour proofing systems employing one or more photosensitive compositions, and comprising an exposure and one or more tranfer steps to a receptor element serving as permanent support. So a system is contemplated by which a colour proof is made by laminating CROMALIN film (registered trade mark of E.I. DU PONT DE NEMOURS Co) to a receptor material, exposing it in contact with a positive or negative selection, removing the cover sheet and toning the tacky image with appropriate colour toner corresponding to a printing ink. Repeating the procedure for each basic colour yields a 4-colour proof. Materials and equipment used in this method are marketed under the trade name CROMALIN by E.I. DU PONT DE NEMOURS Co. Other similar off-press colour proofing systems using four pigmented photosensitive layers which are transferred to a single receptor element are TRANSFER KEY and MATCHPRINT, which are marketed by MMM Co.

It is clear that beside proofing the image carriers of the present invention can be used in any photographic application where control of dot gain or line enlargement is important. So they can be used as carriers for a black-and-white reprographic image, e.g. an image formed upon it by coating, exposing and developing a silver halide emulsion layer, or a silver image formed upon it by the diffusion transfer reversal (DTR) process. The principles of the silver complex diffusion transfer reversal process have been described e.g. in US 2,352,014 and in the book "Photographic Silver Halide Diffusion Processes" by André Rott and Edith Weyde - The Focal Press - London and New York, (1972).

The following examples illustrate the present invention without however limiting it thereto.

### EXAMPLE 1

In this first example the image carriers of the invention serve as permanent supports for multicolour colloid images intended as pre-press colour proofs.

### 1.1. Preparation of control image carriers.

Three different image carriers containing in their hydrophylic binder layer varying amounts of rutile titanium dioxide were prepared (RE-C1-L, RE-C2-M, and RE-C3-H, respectively meant for low, medium and high dot gain). As support of the receptor elements a paper base of 180 g/m was chosen covered on both sides with a polyethylene layer at 30 g/m. A binder layer was coated at a coverage of 1.66 g gelatin/m containing following ingredients per m: 1.4 g of a dispersion of 2.86 % polymethylmethacrylate in a 9.5 % gelatin solution, 0.17 g and 0.34 g respectively of two kinds of silica matting agents, 8.46 g of a dispersion of copoly(methylmethacrylate-styrene, 50/50)-latex in water, 1.1 ml of a 11.7 % saponine solution and 0.12 ml of a 20 % solution of hardener formaldehyde ; the amounts of rutile titanium dioxide were respectively :

| | RE-C1-L | RE-C2-M | RE-C3-H |
|---|---|---|---|
| g rutile-TiO₂/m: | 5.45 | 1.58 | 0.76 |

A fourth control receptor material was prepared (RE-C4-L) was prepared containing essentially the same ingredients as RE-C1-L but with replacement of the rutile-TiO₂ by an equivalent amount of anatase-TiO₂.

### 1.2. Preparation of invention image carriers.

Image carriers according to the invention (RE-I1-L and RE-I2-M, meant for low and medium dot gain respectively) were prepared similar to the control examples but containing following opacifying agents in their hydrophilic binder layer :

| | RE-I1-L | RE-I2-M |
|---|---|---|
| g rutile-TiO₂Mm : | - | 0.25 |
| g anatase-TiO₂/m : | 7.06 | 1.58 |
| g ROPAQUE-OP 62 (solid)/m : | 1.59 | 0.38 |
| (Rohm and Haas) | | |

### 1.3. Evaluation.

The whiteness of the finished image carriers was measured on a GRETAG SMP 100 apparatus, and the resulting colorimetric values are represented in table 1.

**TABLE 1**

| | RE-C1-L | RE-C2-M | RE-C3-H | RE-C4-L | RE-I1-L | RE-I2-M |
|---|---|---|---|---|---|---|
| L∗ | 97.4 | 97.3 | 97.2 | 96.8 | 98.3 | 97.6 |
| a∗ | -0.6 | -0.2 | 0.3 | 0.1 | 0.2 | 0.2 |
| b∗ | 2.1 | 1.1 | 0.1 | 0.3 | 0.2 | -0.1 |

For the evaluation of the percentual dot gain (on a 50 % dot original) a transfer was performed onto the different image carriers serving as receptor elements of respectively cyan, magenta, yellow and black pigmented commercial positive acting AGFAPROOF layers. Each tranferred layer was exposed in a contact-exposure unit using an UV-source through a test original comprising a 50 % dot pattern. Then the exposed samples were processed in a sequence comprising the steps of hardening developing in a hydroquinone - Phenidone developing solution, bleach-fixing in a solution containing Fe(III)-ethylenediamine-tetraacetic-acid and ammonium thiosulphate and a wash-off step using water at 40 °C. The dot gain results are summarized in table 2 :

**TABLE 2**

| pigment | receptor element | | | | | |
|---|---|---|---|---|---|---|
| | RE-C1-L | RE-C2-M | RE-C3-H | RE-C4-L | RE-I1-L | RE-I2-M |
| cyan | 14 | 18 | 22 | 16 | 14 | 18 |
| magenta | 11.5 | 16 | 20 | 13 | 12 | 15.5 |
| yellow | 9.5 | 15 | 19 | 12 | 9 | 14 |
| black | 9 | 14 | 19 | 11 | 9 | 15 |

From the results of table 1 and 2 it is clear that increasing amounts of rutile-TiO₂ (control examples) can give rise to a sufficient low dot gain but the yellowish hue becomes prohibitive. On the other hand, replacing rutile-TiO₂ by the anatase modification - compare control elements RE-C1-L and RE-C4-L - avoids the yellowish hue but the dot gain is unsufficiently low. On the contrary, the receptor elements containing the opacifying agents used in accordance with the present invention show the desired dot gain values without substantially deviating from ideal whiteness. In this way a set of image carriers serving as receptor elements can be provided giving rise to a wide range of attainable dot gains but with essentially the same whiteness.

### EXAMPLE 2

This second example relates to the application wherein the image carrier of the invention serves as image receiving layer for a Diffusion Transfer Reversal (DTR) reprographic image.

### 2.1. Preparation of image receiving layer :

A coating solution containing Ag,Ni-sulphide physical development nuclei was prepared as follows.

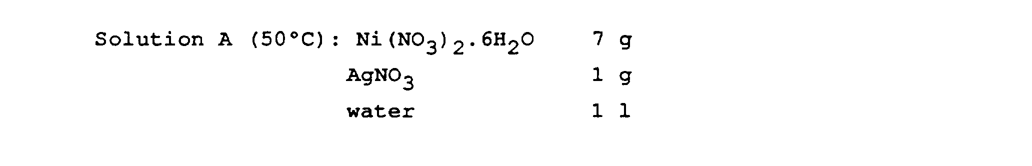

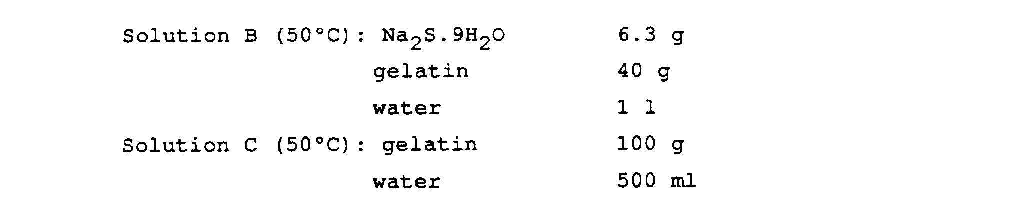

Solution A and B are simultaneously added to solution C at a rate of 100 ml/min. whilst stirring solution C at 400 rpm. After the addition of A and B to C the obtained mixtured was stirred for an additional 5 minutes.

The image receiving material was obtained by applying the described coating solution respectively to image carriers RE-I1-L and RE-I2-M according to the previous example. A top layer was then provided containing 0.7 g of gelatin per m.

### 2.2. Preparation of negative working light sensitive material :

A paper support having a weigth of 110 g/m being covered on both sides with a polyethylene layer was coated at one side with an antihalation layer on the basis of carbon black dispersed in gelatin wherein also hydroquinone and 1-phenyl-4-methyl-pyrazolidin-3-on were present at a coverage of 0.57 g/m and 0.32 g/m. On said antihalation layer an orthochromatically sensitized negative working gelatino silver halide emulsion layer containing an amount of silver chlorobromide (1.8 mol % bromide) equivalent to 2.0 g/m of silver nitrate was coated. The average grain size of the silver chlorobromide was 0.3 microns. The silver halide emulsion layer was overcoated with a thin protective gelatin layer.

A processing solution containing following ingredients was prepared :

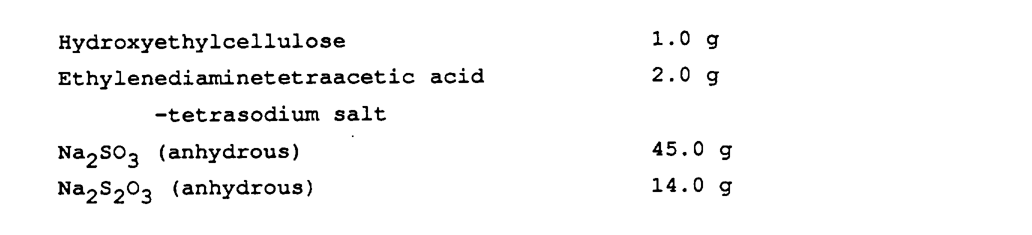

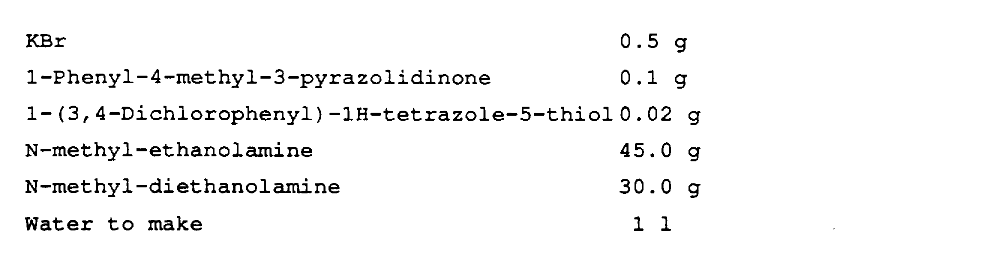

The photographic materials were exposed through an UGRA control wedge in a contact exposure apparatus operating with a light source having a colour temperature of 3200 °K.

### 2.3. DTR-transfer procedure :

The exposed photographic materials were pre-moistened during 6 seconds with the above defined processing liquid, and then pressed together with an image-receiving material as defined above in a conventional DTR-developing solution at 22 °C. The transfer processor employed was a COPYPROOF (registered trade name of AGFA-GEVAERT N.V.) type CP 380. The transfer contact time was 40 seconds.

The obtained test wedge DTR-images on the image-receiving materials were evaluated with regard to maximum density (Dₘₐₓ) and dot gain using a MACBETH densitometer type RD 91858. The results are represented in table 3:

**TABLE 3**

| receptor element : | dot gain | Dmax |
|---|---|---|
| RE-I1-L + nuclei containing layer | 3.5 % | 2.04 |
| RE-I2-M + nuclei containing layer | 12 % | 1.94 |

The variation of the obtained dot gain in dependence on the composition of the image carrier serving as receptor element is obvious from this table.

## Claims

1. Process for the production of a line and/or halftone reprographic image comprising the steps of exposing information-wise a photosensitive composition and transferring it before or after said exposure step to an image carrier comprising a support and an opaque hydrophilic colloid layer, characterized in that said opaque hydrophilic colloid layer contains at least two kinds of opacifying agents, being
(a) polymeric particles consisting of hollow spherical core/sheat beads,
(b) titanium dioxide of the anatase crystal modification.

2. Process according to claim 1 wherein said opaque hydrophilic colloid layer further contains titanium dioxide of the rutile crystal modification.

3. Process according to claim 1 or 2 wherein said line and/or halftone reprographic image is a multicolour colloid pattern formed in said photosensitive composition.

4. Process according to claim 3 wherein said multicolour colloid pattern is formed by the following steps :
(1) transferring a silver halide emulsion layer containing substantially non-hardened gelatin and coloured pigment particles dispersed therein from a temporary support onto said image carrier,
(2) exposing information-wise the silver halide emulsion layer prior to or after carrying out said step (1),
(3) forming by hardening development a halftone image in said transferred silver halide layer and bleach-fixing said layer in order to remove developed silver and undeveloped silver halide,
(4) wash-off processing said transferred layer in order to remove selectively unhardened portions of said layer,
(5) repeating the previous steps in order to form at least partially superposed portions of differently coloured gelatin layers.

5. Process according to claim 1 or 2 wherein said line and/or halftone reprographic image is a black-and-white diffusion transfer reversal image formed by the following steps :
(1) applying an extra colloid layer containing development nuclei to said image carrier ;
(2) preparing a photosensitive negative working silver halide material ;
(3) exposing information-wise said negative working silver halide material;
(4) performing a diffusion transfer reversal step by pressing together in a DTR-developing solution the negative silver halide material and the receptor material provided with said extra colloid layer containing development nuclei.

6. Process according to any of claims 1 to 5 wherein said polymeric particles consisting of hollow spherical core/sheat beads are composed of a copolymer of acrylic acid and styrene.

7. Process according to any of claims 1 to 6 wherein the ratio of the amounts of said opacifying agent (a) and said opacifying agent (b), both expressed as weight, is comprised between 10/1 and 1/10.

8. Process according to any of claims 1 to 7 wherein said support is a paper base covered with a polyolefine layer.

9. Process according to claim 8 wherein said polyolefine layer contains titanium dioxide.

10. Process according to any of claims 1 to 7 wherein said support is an organic polymeric resin support.

11. Process according to claim 10 wherein said organic polymeric resin support contains titanium dioxide.

## Patentansprüche

1. Verfahren zur Herstellung eines reprographischen Strich- und/oder Rasterbilds, das die Schritte der informationsmäßigen Belichtung einer lichtempfindlichen Zusammensetzung und ihrer Übertragung, vor oder nach dem Belichtungsschritt, auf einen Bildträger, der aus einem Träger und einer opaken hydrophilen Kolloidschicht besteht, umfaßt, dadurch gekennzeichnet, daß die opake hydrophile Kolloidschicht mindestens zwei Arten von Trübungsmitteln enthält, nämlich
(a) Polymerteilchen aus hohlen, kugelförmigen Kern-Mantel-Perlen,
(b) Titandioxid der Kristallmodifikation Anatas.

2. Verfahren nach Anspruch 1, bei dem die opake hydrophile Kolloidschicht zusätzlich noch Titandioxid der Kristallmodifikation Rutil enthält.

3. Verfahren nach Anspruch 1 oder 2, bei dem es sich bei dem reprographischen Strich- und/oder Rasterbild um ein in der lichtempfindlichen Zusammensetzung gebildetes mehrfarbiges Kolloidmuster handelt.

4. Verfahren nach Anspruch 3, bei dem man das mehrfarbige Kolloidmuster nacht folgenden Schritten herstellt:
(1) Übertragung einer Silberhalogenidemulsionsschicht, die im wesentlichen ungehärtete Gelatine und darin dispergierte farbige Pigmentteilchen enthält, von einem temporären Träger auf den Bildträger,
(2) informationsmäßige Belichtung der Silberhalogenidemulsionsschicht vor oder nach der Ausführung von Schritt (1),
(3) Bildung eines Rasterbilds in der übertragenen Silberhalogenidschicht durch Härteentwicklung, sowie Bleichfixierung der Schicht zur Entfernung von entwickeltem Silber und nicht entwickeltem Silberhalogenid,
(4) Auswaschverarbeitung der übertragenen Schicht zur selektiven Entfernung ungehärteter Anteile der Schicht,
(5) Wiederholung der vorgenannten Schritte zur Bildung von mindestens teilweise überlagerten Anteilen verschiedenfarbiger Gelatineschichten.

5. Verfahren nach Anspruch 1 oder 2, bei dem es sich bei dem reprographischen Strich- und/oder Rasterbild um ein schwarzweißes Diffusionsübertragungsumkehrbild handelt, das man durch folgende Schritte bildet:
(1) Auftragung einer zusätzlichen, Entwicklungskeime enthaltenden Kolloidschicht auf den Bildträger,
(2) Vorbereitung eines lichtempfindlichen, negativ arbeitenden Silberhalogenidmaterials,
(3) informationsmäßige Belichtung des negativ arbeitenden Silberhalogenidmaterials,
(4) Durchführung eines Diffusionsübertragungsumkehrschritts durch Zusammenpressen des negativen Silberhalogenidmaterials und des mit der zusätzlichen, Entwicklungskeime enthaltenden Kolloidschicht versehenen Empfangsmaterials in einer DTR-Entwicklungslösung.

6. Verfahren nach einem der Ansprüche 1 bis 5, bei dem die aus hohlen, kugelförmigen Kern-Mantel-Perlen bestehenden Polymerteilchen aus einem Copolymer aus Acrylsäure und Styrol bestehen.

7. Verfahren nach einem der Ansprüche 1 bis 6, bei dem das Mengenverhältnis des Trübungsmittels (a) zum Trübungsmittel (b), jeweils bezogen auf das Gewicht, bei 10/1 bis 1/10 liegt.

8. Verfahren nach einem der Ansprüche 1 bis 7, bei dem es sich bei dem Träger um einen mit einer Polyolefinschicht überzogenen Papierträger handelt.

9. Verfahren nach Anspruch 8, bei dem die Polyolefinschicht Titandioxid enthält.

10. Verfahren nach einem der Ansprüche 1 bis 7, bei dem es sich bei dem Träger um einen Träger aus polymerem organischen Harz handelt.

11. Verfahren nach Anspruch 10, bei dem der Träger aus polymerem organischen Harz Titandioxid enthält.

## Revendications

1. Procédé de production d'une image reprographique en ligne et/ou en demi-teinte comprenant les étapes qui consistent à exposer, selon une information, une composition photosensible et à la transférer avant ou après ladite étape d'exposition sur un support d'image comprenant un support et une couche de colloïde hydrophile opaque, caractérisé en ce que ladite couche de colloïde hydrophile opaque contient au moins deux types d'agents opacifiants qui sont:
(a) des particules polymères constituées de billes sphériques creuses à enveloppe et noyau,
(b) du dioxyde de titane à structure cristalline de type anatase.

2. Procédé selon la revendication 1, dans lequel ladite couche de colloïde hydrophile opaque contient, en outre, du dioxyde de titane de structure cristalline de type rutile.

3. Procédé selon la revendication 1 ou 2, dans lequel ladite image reprographique en ligne et/ou en demi-teinte est un dessin de colloïde polychrome formé dans ladite composition photosensible.

4. Procédé selon la revendication 3, dans lequel ledit dessin de colloïde polychrome est formé par les étapes suivantes qui consistent à:
(1) transférer une couche d'émulsion à l'halogénure d'argent contenant de la gélatine pratiquement non durcie et des particules de pigment colorées dispersées dans celle-ci, d'un support temporaire sur ledit support d'image,
(2) exposer, selon une information, la couche d'émulsion à l'halogénure d'argent avant ou après avoir réalisé l'étape (1),
(3) former par un développement avec durcissement une image en demi-teinte dans ladite couche d'halogénure d'argent transférée et fixer par blanchiment ladite couche, pour éliminer l'argent développé et l'halogénure d'argent non développé,
(4) réaliser un lavage de ladite couche transférée pour éliminer sélectivement les parties non durcies de ladite couche,
(5) répéter les étapes précédentes pour former des zones au moins partiellement superposées de couches de gélatine de couleurs différentes

5. Procédé selon la revendication 1 ou 2, dans lequel ladite image reprographique en ligne et/ou en demi-teinte est une image de transfert inverse par diffusion en noir et blanc formée par les étapes suivantes qui consistent à:
(1) appliquer une couche de colloïde supplémentaire contenant des noyaux de développement sur ledit support d'image;
(2) préparer un matériau photosensible, fonctionnant en négatif, à l'halogénure d'argent;
(3) exposer ledit matériau fonctionnant en négatif, à l'halogénure d'argent, selon une information;
(4) réaliser une étape de transfert inverse par diffusion en mettant sous presse ensemble, dans une solution de développement de transfert inverse par diffusion (DTR), le matériau négatif à l'halogénure d'argent et le matériau récepteur recouvert de ladite couche de colloïde supplémentaire contenant des noyaux de développement.

6. Procédé selon l'une quelconque des revendications 1 à 5, dans lequel lesdites particules polymères constituées de billes sphériques creuses à enveloppe et noyau sont constituées d'un copolymère acide acrylique/styrène.

7. Procédé selon l'une quelconque des revendications 1 à 6, dans lequel le rapport des quantités dudit agent opacifiant (a) audit agent opacifiant (b), tout deux exprimés en poids, est compris entre 10/1 et 1/10.

8. Procédé selon l'une quelconque des revendications 1 à 7, dans lequel le support est une base en papier recouverte d'une couche de polyoléfine.

9. Procédé selon la revendication 8, dans lequel ladite couche de polyoléfine contient du dioxyde de titane.

10. Procédé selon l'une quelconque des revendications 1 à 7, dans lequel ledit support est un support en résine polymère organique.

11. Procédé selon la revendication 10, dans lequel ledit support en résine polymère organique contient du dioxyde de titane.
